# EUROPÄISCHE PATENTSCHRIFT

(11) **EP 2 437 046 B1**
(45) Veröffentlichungstag und Bekanntmachung des Hinweises auf die Patenterteilung: **13.08.2014**
(21) Anmeldenummer: 10181729.4
(22) Anmeldetag: 29.09.2010
(51) Int. Cl.: G01N 21/27, G01N 33/00, G01J 3/12, G01N 21/35, G01N 21/03

(54) **Vorrichtung und Verfahren zum Messen von SO3 und H2SO4 Konzentrationen in Gasen**
Device and method for measuring SO3 and H2SO4 concentrations in gases
Dispositif et procédé de mesure de concentrations de SO3 et H2SO4 dans des gaz

(43) Veröffentlichungstag der Anmeldung: 04.04.2012
(73) Patentinhaber: SICK AG, 79183 Waldkirch (DE)
(72) Erfinder: Baasner, Jörn, 88662 Überlingen (DE); Breton, Dr. Heimo, 88690 Uhldingen-Mühlhofen (DE); Schuler, Sonja, 88709 Meersburg (DE)
(74) Vertreter: Ludewigt, Christoph

(56) Entgegenhaltungen:
- US-A- 4 078 896
- US-A- 4 549 080
- US-B1- 6 396 056
- US-B1- 6 486 474
- MAKI A ET AL: "High-resolution infrared spectra of the [nu]2, [nu]3, [nu]4, and 2[nu]3 bands of <32>S<16>O3", JOURNAL OF MOLECULAR SPECTROSCOPY ACADEMIC PRESS USA, Bd. 210, Nr. 2, Dezember 2001 (2001-12), Seiten 240-249, XP002625096, ISSN: 0022-2852
- GIVAN A ET AL: "Matrix isolation mid- and far-infrared spectra of sulfuric acid and deuterated sulfuric acid vapors", JOURNAL OF MOLECULAR STRUCTURE ELSEVIER NETHERLANDS, Bd. 509, 12. Oktober 1999 (1999-10-12), Seiten 35-47, XP002625097, ISSN: 0022-2860
- RAWLINS W T ET AL: "QUANTUM CASCADE LASER SENSOR FOR SO2 AND SO3 FOR APPLICATION TO COMBUSTOR EXHAUST STREAMS", APPLIED OPTICS, OPTICAL SOCIETY OF AMERICA, WASHINGTON, DC; US, vol. 44, no. 31, 1 November 2005 (2005-11-01), pages 6635-6643, XP001236488, ISSN: 0003-6935, DOI: 10.1364/AO.44.006635
- PAUL E. HINTZE ET AL: 'Vibrational and Electronic Spectroscopy of Sulfuric Acid Vapor' THE JOURNAL OF PHYSICAL CHEMISTRY A Bd. 107, Nr. 8, 01 Februar 2003, Seiten 1112 - 1118, XP055114057 DOI: 10.1021/jp0263626 ISSN: 1089-5639

## Beschreibung

Die Erfindung betrifft eine Vorrichtung und ein Verfahren zum Messen von SO₃ und/oder H₂SO₄ Konzentrationen in Gasen.

NOₓ Emissionen in Verbrennungsgasen, z. B. in Kohlekraftwerken oder sonstigen Feuerungsanlagen, sind durch gesetzliche Auflage stark beschränkt, so dass häufig die sogenannte Selektive-Katalytische-Reduktion (SCR) Technologie angewendet werden muss. Eine Folge davon ist die Oxidation von SO₂ zu SO₃ in dem Verbrennungsgas. Eine verstärkte SO₃ Emission hat aber schwerwiegende Nachteile wie zum Beispiel eine verstärkte Neigung zur Bildung von AmmoniumBisulfat im Lufterhitzer, Korrosionsprobleme von Oberflächen schon bei Temperaturen unterhalb des Säuretaupunktes und verstärkte Lufttrübung und Schwadenbildung durch Säure-Aerosole. Um die Menge SO₃ zu kontrollieren, werden häufig Additive in das Abgas injiziert. Die Injektionsrate muss jedoch kontrolliert werden und hängt von der Konzentration von SO₃ und gebildetem H₂SO₄ ab. Derzeit ist kein Verfahren und Vorrichtung bekannt, mit der SO₃ und/oder H₂SO₄ Konzentrationen kontinuierlich messbar sind. Bisherige Messungen erfolgen manuell oder nicht-kontinuierlich mittels FTIR-Spektroskopie. Die Möglichkeit einer kontinuierlichen SO₃ Messung würde eine große Kostenersparnis bedeuten bei gleichzeitig minimalem Einsatz von Additiven zur Kontrolle der SO₃ Emissionen. Selbst wenn Additive nicht gebraucht werden, kann eine genaue und kontinuierliche SO₃ Messung dazu dienen, den SO₃ Gehalt zu verfolgen, um Anstiege des SO₃ Gehalts zu erkennen bei Änderungen in der Brennstoffzusammensetzung oder Änderung des SCR-Betriebs, so dass entsprechende Korrekturmaßnahmen ergriffen werden können. Eine kontinuierliche SO₃ Beobachtung würde auch in Kraftwerken, die schwefelhaltigen Kohle verbrennen, helfen, den Gehalt an SO₃ im Abgas zu reduzieren.

Aus der US 6,486,474 B1 und der US 4,078,896 sind prinzipiell Photometer bekannt. Mit diesen sind in dem einen Fall Isotopenverhältnisse von CO₂ und in dem anderen Fall Konzentrationen von SO₂, NO, NO₂ und H₂S bestimmbar.

Aus Journal of Molecular Spectroscopy 210, 240-249 (2001) sind Absorptionsspektren für SO₃ und aus Journal of Molecular Structure 509 (1999), 35-47 Absorptionsspektren für H₂SO₄ bekannt.

Aus der US 6,396,056 B1 ist ein Gasanalysator bekannt, mit dem die Konzentration von SO₃ und H₂SO₄ bestimmbar ist. Dieses Gerät arbeitet nach dem in-situ Prinzip. Zur Kalibirierung wird eine Referenzgaszelle ("audit-cell" genannt) in den Strahlengang eingeschoben, was die Kalibirierung am Einbauort erlaubt.

Es ist Aufgabe der Erfindung, eine verbesserte Vorrichtung und Verfahren bereitzustellen, mit der eine kontinuierliche Messung der SO₃ und H₂SO₄ Konzentrationen in einem Gas wirtschaftlich möglich ist.

Diese Aufgabe wird gelöst durch eine Vorrichtung mit den Merkmalen des Anspruchs 1 und ein Verfahren mit den Merkmalen des Anspruchs 5.

Die erfindungsgemäße Vorrichtung zum kontinuierlichen Messen von SO₃ und/oder H₂SO₄ Konzentrationen in Gasen umfasst ein Photometer mit einer Lichtquelle, einer Küvette, der das zu messende Gas über einen Gaseinlass zugeführt und über einen Gasauslass abgeführt wird, einem Empfänger, einer optischen Filtereinheit mit wenigstens einem optischen Filter, das derart ausgewählt ist, dass es eine Messwellenlänge durchlässt, die derart gewählt ist, dass sie von SO₃ und/oder H₂SO₄ möglichst gut und von den anderen Komponenten des Gases möglichst wenig absorbiert wird. Das Photometer wurde mit SO₃ und H₂SO₄ Gasen bekannter Konzentration kalibriert. Es umfasst weiter eine Auswerteeinrichtung, die eine Speichereinrichtung umfasst, in der bereits vermessene Querempfindlichkeiten bei der Messwellenlänge in Tabellen hinterlegt sind. Aus den Photosignalen und den hinterlegten Querempfindlichkeiten kann die Auswerteeinrichtung einen Konzentrationswert für SO₃ und/oder H₂SO₄ kontinuierlich ermitteln.

Mit der Erfindung ist erstmalig eine Vorrichtung und ein Verfahren bereitgestellt, mit der kontinuierlich S03 und H2S04 gemessen werden kann. Dabei hat sich herausgestellt, dass dies mit einem einfachen Photometer möglich ist, wenn eine geeignete Messwellenlänge genommen wird und ein entsprechendes Filter bereitgestellt wird, wobei insbesondere alle Querempfindlichkeiten berücksichtigt werden. Die Querempfindlichkeiten müssen im Vorhinein bestimmt werden und sind dann aus einer Speichereinrichtung, in der sie in Tabellen abgelegt sind, abrufbar. Somit kann aus dem gemessenen Photostrom bei der Messwellenlänge unter Berücksichtigung der Querempfindlichkeiten die SO₃ bzw. H₂SO₄ Konzentration bestimmt werden. Eine kontinuierliche Bestimmung bedeutet dabei, dass entweder permanent der Photostrom auf der Messwellenlänge gemessen wird oder aber auch, dass zyklisch auch andere Gaskomponenten durch Wechseln des Filters, beispielsweise mittels eines Filterrades, erfasst werden und deren Konzentration ermittelt wird. Typischerweise kann ein solcher Zyklus mehrmals pro Minute durchlaufen, so dass jede Komponente "quasi-kontinuierlich" bestimmt wird.

Durch die Möglichkeit jetzt kontinuierlich SO₃ und H₂SO₄ messen zu können, können die eingangs genannten Nachteile vermieden werden.

Ein ganz wesentlicher Aspekt der Erfindung ist, dass die Vorrichtung vor dem Einsatz mit SO₃ und H₂SO₄ Gasen bekannter Konzentration kalibriert wurde. Dies ist keine selbstverständlich Maßnahme, denn SO₃ und H₂SO₄ Spektren sind in der Literatur nur unvollständig erhältlich. Das liegt auch daran, dass SO₃ bei Anwesenheit von Wasser sofort zu H₂SO₄ reagiert und H₂SO₄ selbst äußerst aggressiv ist.

Für fast alle Verbrennungsgase hat sich herausgestellt, dass als Messwellenlänge für SO₃ eine Wellenlänge, die zwischen 7050 und 7250 nm liegt, geeignet ist und für H₂SO₄ zwischen 10800 und 12200 nm. In diesen Wellenlängenbereichen ist eine solche Absorption vorhanden, die bei Berücksichtigung der Querempfindlichkeiten gute Ergebnisse liefert.

In Weiterbildung der Erfindung weist das Fotometer einen optischen Justagefilter auf, der auf den Messwellenlängen eine konstante, bekannte Absorption erzeugt. Der Justagefilter dient in erster Linie dazu, Drifts, wie zum Beispiel Temperaturdrifts, zu erkennen und entsprechende Korrekturfaktoren bereitzustellen, so dass das Messergebnis letztendlich unabhängig von derartigen Drifts ist. Die Nachjustage und Driftüberwachung wird in bestimmten Zeitabständen durch eine Justagemessung bevorzugt automatisch vorgenommen.

Das erfindungsgemäße Verfahren zum kontinuierlichen Messen von SO₃ und/oder H₂SO₄ Konzentrationen in Gasen umfasst die Schritte:
- Erzeugen eines SO₃ und/oder H₂SO₄ Absorptionsspektrums,
- Auswahl einer Messwellenlänge die für SO₃ zwischen 7050 und 7250 nm und für H₂SO₄ zwischen 10800 und 12200 nm liegt,
- Bereitstellen eines optischen Filters für die Messwellenlänge,
- Kalibrieren eines Photometers bei der Messwellenlänge mit Gasen bekannter Konzentration,
- Zu- und Abführen des zu messenden Gases zu und von einer Küvette,
- Erfassen von Querempfindlichkeiten an der Messwellenlänge gegenüber allen in dem zu messenden Gas vorkommenden Komponenten,
- Hinterlegen der Querempfindlichkeiten in einer Speichereinheit einer Auswerteeinrichtung für das Photometer,
- Messen der Absorption des Gases mit dem Photometer bei der Messwellenlänge,
- Korrigieren der gemessenen Absorptionswerte mit den hinterlegten Querempfindlichkeiten
- und Ermitteln der Konzentration von SO₃ und/oder H₂SO₄ aus den korrigierten Absorptionswerten.

Im Folgenden wird die Erfindung anhand eines Ausführungsbeispiels unter Bezugnahme auf die Zeichnung im Einzelnen erläutert. In der Zeichnung zeigen:

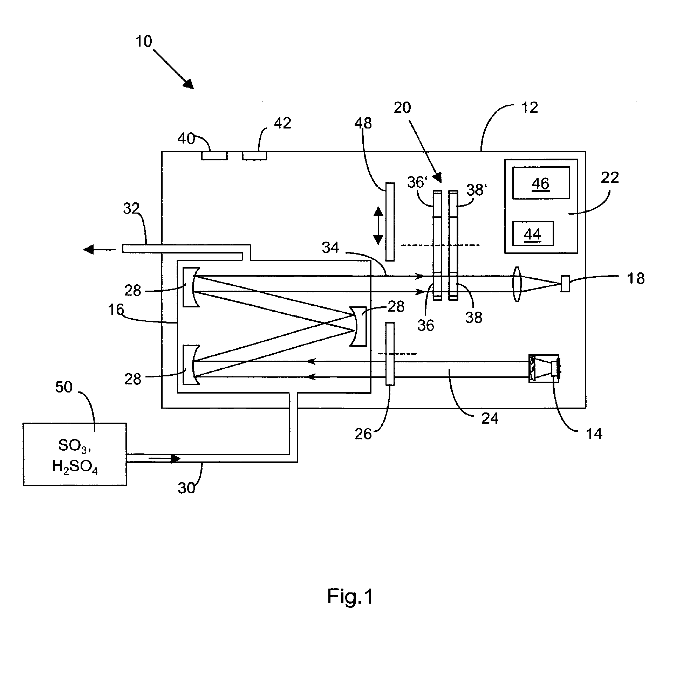

Fig.1 eine erfindungsgemäße Vorrichtung;

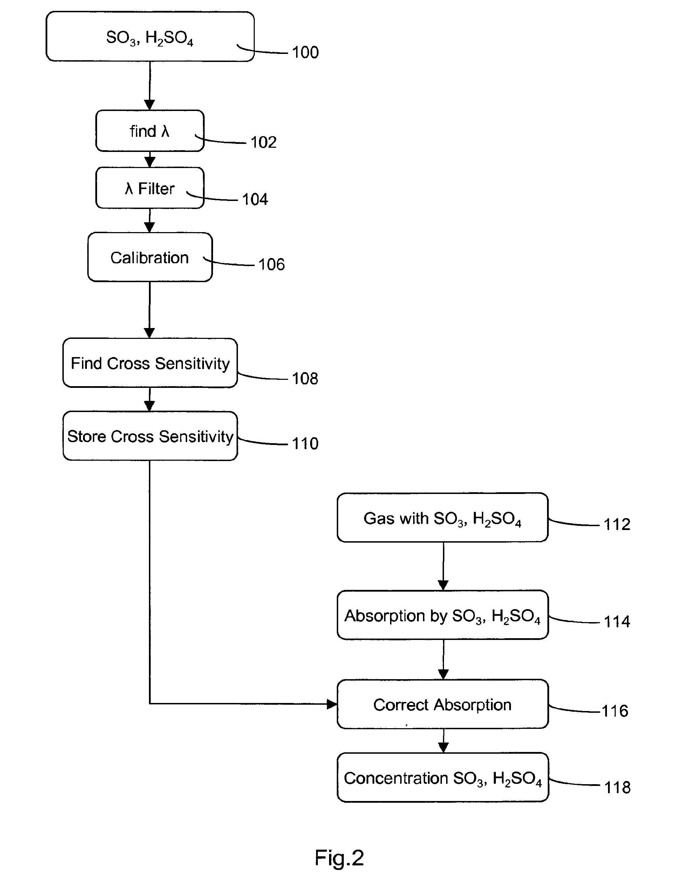

Fig.2 wesentliche Schritte des erfindungsgemäße Verfahrens.

Eine erfindungsgemäße Vorrichtung ist als Fotometer 10 ausgebildet. Das Fotometer 10 umfasst in einem Gehäuse 12 eine Lichtquelle 14, eine Küvette 16, einen Lichtempfänger 18, eine optische Filtereinheit 20 und eine Auswerteeinrichtung 22.

Das Fotometer 10 ist als Einstrahlinfrarotfilterfotometer ausgebildet, das den gleichzeitigen Einsatz von Bifrequenz und Gasfilterkorrelationsverfahren erlaubt. Die Lichtquelle 14 sendet infrarotes Licht 24 aus und zeichnet sich durch eine hohe Energieabgabe und eine lange Lebensdauer aus. Das gesendete Licht 24 durchläuft ein Chopperrad 26 und tritt in die Küvette 16 ein.

Die Küvette 16 weist eine große optische Weglänge und ein kleines Volumen auf. Die optische Weglänge der Küvette 16 ist fest über in die Stirnseiten eingefräste Spiegel 28 eingestellt und beträgt je nach Auslegung 3 oder 6 m. Die Küvette 16 ist auf geringes Volumen und schnellen Gasaustausch hin optimiert. Das zu messende Gas wird über einen Gaseinlass 30 der Küvette 16 zugeführt und über einen Gasauslass 32 abgeführt. Sie kann auf Temperaturen bis zu 220°C eingestellt werden. Im Gaseingang befindet sich ein Schutzfilter. Optional kann ein nicht dargestellter Durchflussmesser zur Strömungsüberwachung des Messgases integriert sein. Alle vom Messgas berührten Teile sind hoch beheizbar, um eine Taupunktunterschreitung zu verhindern.

Dass die Küvette 16 verlassende Licht 34 durchläuft die Filtereinheit 20 mit ihren Filtern 36 und 38, bei denen es sich um Interferenz- bzw. Gasfilter handelt und trifft auf den Lichtempfänger 18, der bevorzugt als pyroelektrischer Detektor ausgebildet ist. Die Filtereinheit 20 umfasst unter anderem einen IR-Filter mit einer Filterwellenlänge, die zwischen 7050 nm und 7250 nm liegt, also dessen Durchlässigkeit mit einer gewissen Halbwertsbreite bei einer bestimmten Filterwellenlänge in diesem Wellenlängenbereich liegt sowie einen weiteren IR-Filter mit einer Filterwellenlänge zwischen 10800 und 12200 nm.

Der Detektor 18 ist mit der Auswerteeinrichtung 22 verbunden, in der das Signal des Detektors 18 ausgewertet wird und die Konzentration von SO₃ bzw. H₂SO₄ im Messgas ermittelt wird. Die Auswerteeinrichtung umfasst dazu einen Steuerrechner, eine Speichereinrichtung 44 und eine Benutzerschnittstelle 46 mit Tastatur und Bildschirm. Über Schnittstellen 40 und 42 können die Messwerte nach außen gegeben werden.

Des Weiteren ist ein optischer Nachjustagefilter 48 vorgesehen, der bei Bedarf zur Nachjustage und Driftüberwachung in den Strahlengang eingeschoben werden kann. Der optionale Einsatz des Nachjustagefilters 48 erlaubt auch die schnelle Kontrolle der eingestellten Empfindlichkeit.

Das erfindungsgemäße Verfahren läuft in folgenden Schritten ab.

Zunächst wird im Schritt 100 SO₃ und H₂SO₄ in verschiedenen Konzentrationen in der Gasphase in einer geeigneten Vorrichtung 50 erzeugt. Das ist mit besonderen Schwierigkeiten verbunden, denn SO₃ ist nicht stabil und kann deshalb nicht käuflich erworben werden und H₂SO₄ ist hochaggressiv und hochkorrosiv. Das genaue Verfahren zur Herstellung der Substanzen ist aber nicht Gegenstand dieser Erfindung und soll deshalb nicht weiter erläutert werden. Mit den Substanzen werden schließlich Absorptionsspektren mit einem FTIR-Spektrometer aufgenommen.

Damit ist es möglich, im Schritt 102 eine Messwellenlänge für SO₃ bzw. H₂SO₄ auszuwählen, bei der die Absorptionsmessungen vorgenommen werden sollen. Es wird eine Wellenlänge ausgewählt, die von SO₃ bzw. H₂SO₄ möglichst gut und von den anderen Komponenten des Messgases möglichst wenig absorbiert wird, um möglichst geringe Querempfindlichkeiten zu haben. Für fast alle Verbrennungsgase hat sich herausgestellt, dass eine Messwellenlänge für SO₃ im Bereich von 7050 bis 7250 nm geeignet ist und für H₂SO₄ eine Wellenlänge zwischen 10800 und 12200 nm. Dann werden im Schritt 104 optische Filter für diese Messwellenlängen, die gegebenenfalls gesondert hergestellt werden müssen, bereitgestellt.

Schließlich wird im Schritt 106 ein Photometer bei diesen Messwellenlängen kalibriert.

Dann werden im Schritt 108 die Querempfindlichkeiten an der Messwellenlänge gegenüber allen in dem zu messenden Gas vorkommenden Komponenten mit dem Fotometer 10 ermittelt und in der Speichereinrichtung 44 in Form von Tabellen abgespeichert (im Schritt 110).

Schließlich wird im Schritt 112 und 114 die Absorption des Messgases mit dem Fotometer bei der Messwellenlänge gemessen, um damit die Absorption durch SO₃ bzw. H₂SO₄ zu bestimmen und daraus im Schritt 118 die Konzentration zu ermitteln. Für die korrekte Konzentrationsbestimmung muss allerdings im Schritt 116 die gemessene Absorption mit den in der Speichereinrichtung hinterlegten Querempfindlichkeiten korrigiert werden, was in der Auswerteeinrichtung erfolgt. Daraus ergeben sich am Ende im Schritt 118 die Werte für die Konzentration von SO₃ und H₂SO₄ aus den korrigierten Absorptionswerten.

Damit bei der Durchleitung des Messgases durch die Küvette keine Kondensation auftritt, muss für eine durchgängige Beheizung auf ca. 200°C aller Komponenten (Messgasentnahmesonde, Messgasfilter, Messgasleitung, Messgaspumpe, Messküvette) Sorge getragen werden. Kältebrücken müssen vermieden werden.

Zur routinemäßigen Überprüfung des laufenden Fotometers kann der Justagefilter in den optischen Strahlengang eingebracht werden. Damit können Justageunrichtigkeiten und Drifts, wie Temperaturdrifts erkannt und in der Auswertung berücksichtigt werden.

Neben den Filtern 36 und 38 weist die Filtereinheit 20 auch andere Filter 36' und 38' auf, bei denen es sich ebenfalls um Interferenz- bzw. Gasfilter handelt, die aber andere Filterwellenlängen aufweisen. Und zwar Wellenlängen, bei denen andere zu messende Gaskomponenten des Messgases absorbieren und vermessen werden. So lassen sich auch Konzentration von z. B. H₂O, NO, SO₂, CO₂ und CH₄ bestimmen. Dazu werden normalerweise Filterräder der Filtereinheit 20 gedreht, so dass die Messung einer Gaskomponenten nur Millisekunden dauert, so dass insgesamt alle gewünschten Gaskomponenten, einschließlich SO₃ und H₂SO₄, (quasi) kontinuierlich gemessen werden.

## Patentansprüche

1. SO₃- und H₂SO₄-Messvorrichtung zum kontinuierlichen Messen von SO₃ und/oder H₂SO₄ Konzentrationen in Gasen mit einem Photometer mit einer Lichtquelle, einer Küvette, der das zu messende Gas über einen Gaseinlass zugeführt und über einen Gasauslass abgeführt wird, einem Empfänger, einer optischen Filtereinheit mit wenigstens einem optischen Filter, das derart ausgewählt ist, dass es eine Messwellenlänge durchlässt, die für SO₃ zwischen 7050 und 7250 nm und für H₂SO₄ zwischen 10800 und 12200 nm liegt und das Photometer mit SO₃ und H₂SO₄ Gasen bekannter Konzentration kalibriert wurde und mit einer Auswerteeinrichtung, die eine Speichereinrichtung umfasst, in der bereits vermessene Querempfindlichkeiten bei der Messwellenlänge gegenüber allen in dem zu messenden Gas vorkommenden Komponenten hinterlegt sind und die Auswerteeinrichtung aus den Photosignalen und den hinterlegten Querempfindlichkeiten einen Konzentrationswert für SO₃ und/oder H₂SO₄ kontinuierlich ermitteln kann.

2. Vorrichtung nach einem der vorhergehenden Ansprüche, **gekennzeichnet durch** einen optischen Justagefilter, der auf den Messwellenlängen eine konstante, bekannte Absorption erzeugt.

3. Verfahren zum kontinuierlichen Messen von SO₃ und/oder H₂SO₄ Konzentrationen in Gasen mit den Schritten:
- Erzeugen eines SO₃ und/oder H₂SO₄ Absorptionsspektrums,
- Auswahl einer Messwellenlänge die für SO₃ zwischen 7050 und 7250 nm und für H₂SO₄ zwischen 10800 und 12200 nm liegt,
- Bereitstellen eines optischen Filters für die Messwellenlänge,
- Kalibrieren eines Photometers bei der Messwellenlänge,
- Zu- und Abführen des zu messenden Gases zu und von einer Küvette,
- Erfassen von Querempfindlichkeiten an der Messwellenlänge gegenüber allen in dem zu messenden Gas vorkommenden Komponenten,
- Hinterlegen der Querempfindlichkeiten in einer Speichereinheit einer Auswerteeinrichtung für das Photometer,
- Messen der Absorption des Gases mit dem Photometer bei der Messwellenlänge,
- Korrigieren der gemessenen Absorptionswerte mit den hinterlegten Querempfindlichkeiten
- und Ermitteln der Konzentration von SO₃ und/oder H₂SO₄ aus den korrigierten Absorptionswerten.

4. Verfahren nach Anspruch 3, **dadurch gekennzeichnet, dass** mit dem Photometer auch andere Gaskomponenten zyklisch erfasst und deren Konzentration ermittelt wird.

5. Verfahren nach einem der vorhergehenden Ansprüche 3 bis 4, **dadurch gekennzeichnet, dass** zur Justage bzw. Driftüberwachung in bestimmten Zeitabständen ein optischer Justagefilter eingesetzt wird, der auf den Messwellenlängen eine konstante, bekannte Absorption erzeugt.

## Claims

1. S03 and H2SO4 measuring device for continuously measuring of S03 and/or H2SO4 concentrations in gases with a photometer comprising a light source, a cuvette, to which the gas to be measured is supplied to via a gas inlet and removed via a gas outlet, a receiver, an optical filter unit with at least one optical filter is selected such that it transmits a measuring wavelength, which lies between 7050 and 7250 nm for S03 and between 10800 and 12200 nm for H2SO4, and the photometer was calibrated with S03 and H2SO4 gases of known concentration, and comprising an evaluation that includes a memory device storing already measured cross-sensitivities over all components occurring in the measured gas at the measuring wavelength, and the evaluation unit can detect a concentration value for S03 and/or H2SO4 continuously from the photo signals and the stored cross-sensitivities.

2. A device according to one of the preceding claims, **characterized by** an optical filter for adjustments that generates a constant and known absorption at the measuring wavelengths.

3. Method for continuously measuring of S03 and/or H2SO4 concentrations in gases comprising the steps of:
- Create an S03 and/or H2SO4 absorption spectrum,
- Selection of a measuring wavelength which for S03 lies between 7050 and 7250 nm and for H2SO4 between 10800 and 12200 nm,
- Providing an optical filter for the measuring wavelength,
- Calibration of a photometer at the measuring wavelength,
- Supply and Removal of the gas to be measured to and from a cuvette,
- Capture of cross-sensitivities at the measuring wavelength over all occurring components in the gas to be measured,
- Storing the cross-sensitivities in a memory device of an evaluation unit for the photometer,
- Measuring the absorption of the gas with the photometer at the measuring wavelength,
- Correcting the measured absorption values with the stored cross-sensitivities,
- and Determining the concentration of S03 and/or H2SO4 from the corrected absorption values.

4. A method according to claim 3, **characterized in that** the photometer detects other gas components and their concentration is determined cyclically.

5. A method according to any one of the preceding claims 3 to 4, **characterized in that** for adjustment or drift monitoring at certain time intervals an optical filter for the adjustments is used which generates a constant known absorption at the measuring wavelengths.

## Revendications

1. S03 et H2SO4 dispositif de mesure pour la mesure en continu des concentrations de S03 et/ou de H2SO4 à gaz avec un photomètre comprenant une source lumineuse, une cuvette, dans laquelle le gaz à mesurer est appliqué à l'intermédiaire d'une entrée de gaz et évacué par l'intermédiaire d'un sortie de gaz, un récepteur, une unité de filtre optique avec au moins un filtre optique est choisi de telle sorte qu'elle transmet une longueur d'onde de mesure, qui se situe entre 7050 et 7250 nm pour S03 et entre 10800 et 12200 nm pour H2SO4, et le photomètre était étalonné avec des gaz S03 et H2SO4 de concentration connue, et comprenant une unité d'évaluation qui comprend une mémoire enregistrée sensibilités transversales déjà mesurés sur l'ensemble de composants présent dans le gaz à mesurée à la longueur d'onde de mesure, et l'unité d'évaluation peut détecter une valeur de concentration pour S03 et/ou de H2SO4 en continu à partir des signaux de photo et les sensibilités transversales enregistrées.

2. Dispositif selon l'une des revendications précédentes, **caractérisé par** un filtre optique pour des ajustements qui génère une absorption constante et connue aux longueurs d'onde de mesure.

3. Procédé pour la mesure en continu des concentrations en gaz S03 et/ou H2SO4, comprenant les étapes consistant à:
- Créer un spectre d'absorption S03 et/ou de H2SO4,
- Sélection de la longueur d'onde de mesure qui pour S03 se situe entre 7050 et 7250 nm et pour H2SO4 entre 10800 et 12200 nm,
- Fourniture d'un filtre optique pour la longueur d'onde de mesure,
- Etalonnage d'un photomètre à la longueur d'onde de mesure,
- Approvisionnement et évacuation du gaz à mesurer à une cuvette et à partir de la cuvette,
- Capture des sensibilités transversales à la longueur d'onde de mesure sur tous les composants qui se produisent dans le gaz à mesurer,
- Enregistrer des sensibilités transversales dans un dispositif de mémoire d'une unité d'évaluation pour le photomètre,
- Mesure de l'absorption du gaz avec le photomètre à la longueur d'onde de mesure,
- Correction des valeurs d'absorption mesurées avec les sensibilités croisées enregistrées,
- Détermination de la concentration et de S03 et/ou de H2SO4 à partir des valeurs corrigées d'absorbtion.

4. Procédé selon la revendication 3, **caractérisé en ce que** le photomètre détecte d'autres composants de gaz et leur concentration est déterminée de manière cyclique.

5. Procédé selon l'une quelconque des revendications précédentes 3-4, **caractérisé en ce que** pour le réglage ou la dérive de surveillance à des intervalles de temps, un filtre optique pour les ajustements est utilisé qui produit une absorption connue constante aux longueurs d'onde de mesure.
